**Europäisches Patentamt**

**European Patent Office**

**Office européen des brevets**

(11) Veröffentlichungsnummer: **0 028 374**
**B1**

(12) **EUROPÄISCHE PATENTSCHRIFT**

(45) Veröffentlichungstag der Patentschrift:
**16.03.83**

(21) Anmeldenummer: **80106514.5**

(22) Anmeldetag: **24.10.80**

(51) Int. Cl.³: **A 23 L 1/30,** A 61 K 35/78,
A 23 L 1/04

(54) Diätmittel, das zur Verringerung des Körpergewichts und Senkung des Serumlipidspiegels geeignet ist, und Verfahren zu seiner Herstellung.

(30) Priorität: **05.11.79 DE 2944535**

(43) Veröffentlichungstag der Anmeldung:
**13.05.81 Patentblatt 81/19**

(45) Bekanntmachung des Hinweises auf die Patenterteilung:
**16.03.83 Patentblatt 83/11**

(84) Benannte Vertragsstaaten:
**AT BE CH DE FR GB IT LI LU NL SE**

(56) Entgegenhaltungen:
**DE-A-2 626 734**
**DE-A-2 629 773**
**DE-A-2 834 227**
**DE-A-2 837 294**

(73) Patentinhaber: **Biotest-Serum-Institut GmbH,
Flughafenstrasse 4, D-6000 Frankfurt-Niederrad (DE)**

(72) Erfinder: **Lissner, Reinhard, Dr. Dipl.-Chem.,
Lippmanweg 23, D-6101 Lichtenberg (DE)**
Erfinder: **Bonhard, Klaus, Dr. Dipl.-Chem.,
Sandeldamm 16, D-6450 Hanau (DE)**
Erfinder: **Bittermann, Karl-Heinz, Bruchstrasse 5,
D-6057 Dietzenbach (DE)**

(74) Vertreter: **Beil, Walter, Dr. et al, BEIL, WOLFF & BEIL
Rechtsanwälte Adelonstrasse 58, D-6230 Frankfurt am
Main 80 (DE)**

Diätmittel, das zur Verringerung des Körpergewichts und Senkung des Serumlipidspiegels geeignet ist, und Verfahren zu seiner Herstellung

Die Erfindung betrifft ein Diätmittel, das zur Verringerung des Körpergewichts und Senkung des Serumlipidspiegels geeignet ist, enthaltend Pektin und Kleie, das dadurch gekennzeichnet ist, dass es neben Pektin und Kleie Gelatine enthält, sowie ein Verfahren zu seiner Herstellung, das dadurch gekennzeichnet ist, dass man Gelatine und Pektin in wenig Wasser löst, in der viskosen Lösung die Kleie und gegebenenfalls die weiteren Zusatzstoffe verteilt und die breiige Masse zu beliebigen Formkörpern verarbeitet.

Es ist bekannt, dass Pektine, insbesondere solche aus Früchten, einen erhöhten Cholesterinspiegel senken können. Es ist auch bereits eine sogenannte «Cholesterin-Minusdiät» bekannt, die Apfelpektin und Vollsojamehl enthält. Dieses bekannte Diätmittel weist einen verhältnismässig hohen Fettgehalt von 11 Gew.-% auf und ist darüberhinaus wegen seiner Pulverform schwierig anzuwenden.

Es ist andererseits bekannt, dass Ballaststoffe, die auch als «Nahrungsfasern» bezeichnet werden und zu denen insbesondere Kleie, aber auch das Pektin gehören, die Fähigkeit besitzen, die Darmtätigkeit anzuregen, und somit eine wichtige Rolle für eine Gewichtsreduktion (über eine natürliche Verminderung des Hungergefühls) und Verhütung von Obstipation spielen können.

Aus der DE-A-26 29 773 ist ein Produkt aus Kleie und Pektin bekannt, das als Heilmittel, diätetisches Nahrungsmittel oder als Snack-Artikel verwendet werden sollte. Soweit dieses bekannte Produkt als diätetisches Nahrungsmittel eingesetzt werden sollte, war es nur als Ergänzung zur üblichen pflanzenfaserarmen Nahrung vorgesehen, da sowohl Kleie als auch Pektin nur teilweise verdaulich sind und allein wegen Unterschreitung des «Eiweissminimum» keinen Ersatz für Hauptmahlzeiten bieten konnten. Das bekannte Produkt sollte zwar gegebenenfalls Ölsaatprodukte oder Getreideprodukte als weiteren Bestandteil enthalten; diese zusätzlichen Bestandteile stellten aber auch keine geeignete Eiweissquelle dar. Das bekannte Produkt aus Kleie und Pektin liess sich zwar besser einnehmen als die beiden Einzelbestandteile, stellte aber für den Verbraucher noch keineswegs ein angenehm einzunehmendes Produkt dar, das bereitwillig angenommen wurde. Beispielsweise «rutschte» es immer noch nicht besonders gut. Darüberhinaus war es auch nicht vielseitig verarbeitbar, und zwar weder im Hinblick auf die Form noch im Hinblick auf die Geschmacksrichtung. Aufgrund dieser relativen Gleichförmigkeit ist das bekannte Produkt auch nicht besonders attraktiv für den Verbraucher.

Der Erfindung liegt die Aufgabe zugrunde, ein Diätmittel bereitzustellen, das bereitwillig angenommen wird und bei täglicher Einnahme, beispielsweise als Ersatz von zwei Hauptmahlzeiten, bei Patienten mit Übergewicht und anderen Risikofaktoren, die für bestimmte Blutgefässerkrankungen, z.B. Myokardinfarkt und Arteriosklerose, prädisponieren, zu folgenden therapeutisch erwünschten Wirkungen führt:

1. Verringerung des Körpergewichts,
2. Senkung eines erhöhten Serumcholesterinspiegels,
3. verbesserte Ausscheidung von Stoffwechselprodukten (z.B. bestimmte Gallensäurederivate), die kanzerogene Wirkungen aufweisen sollen, mit dem Stuhl,
4. Prophylaxe von Divertikulose des Darms und
5. allgemeine Verbesserung von Verdauungsvorgängen.

Diese Aufgabe wird durch das erfindungsgemässe Diätmittel in überraschend befriedigender Weise gelöst.

Durch die Verwendung von Gelatine als Träger für die Komponenten Kleie und Pektin werden gegenüber dem bekannten Produkt aus Kleie und Pektin die folgenden überraschenden Vorzüge erreicht:

1. Gelatine ist ein günstiger Nahrungsquellen-Lieferant bestimmter Aminosäuren, die der menschliche Organismus täglich benötigt und ohne deren Bereitstellung ein gesundheitsschädigender kataboler Effekt bei über 10tägiger Einnahme eines Kleie-Pektingemisches eintritt («Eiweissminimum»). Allgemein wird eine tägliche Zufuhr von 1 g tierischem Protein pro kg Körpergewicht als Eiweissoptimum angesehen.
2. Durch die Bereitstellung der Aminosäuren aus der Gelatine wird ein Einfluss auf die zentralnervösen Stoffwechselzentren im Sinne einer Grundumsatzsteigerung («spezifisch dynamische Steigerung») ausgeübt, der durch eine Steigerung auch von lipolytischen Vorgängen die niederkalorische Diät hinsichtlich einer Körpergewichtsreduktion wirkungsvoll unterstützt.
3. Durch die besondere Quellfähigkeit der Gelatine wird der Tonus der Magen-Darmwand überraschend erhöht und das Nichtaufkommen von Hungergefühl weiter überraschend günstig beeinflusst.
4. Die Einarbeitung der Gelatine als Eiweiss- und Quellbestandteil erlaubt eine Verarbeitung der Wirkkomponenten zu geschmacksneutralen und technisch besonders vielseitig aufarbeitbaren Produkten, deren Konsistenz und Aussehen überraschend ansprechend sind. Die Masse kann nach allen Geschmacksrichtungen hin aromatisiert werden, z.B. mit Frucht-, Kräuter-, Käse-, Gemüse-, Fleisch- und Nuss-Aroma. Der Geschmack wird als abgerundet empfunden. Die Masse kann geschäumt oder fadenförmig extrudiert und zu Geflechten versponnen, dann getrocknet, tafelförmig gepresst oder granuliert und abermals gepresst werden. Jeweils dem Charakter des Aromas entsprechend lässt sich das wirksame Basisgemisch in eine feucht-fruchtige oder knusprig-bröckelige oder faserige oder geschichtete blättrige Konsistenz bringen. Aus diesen Varianten lässt sich eine Vielfalt von Menüs, z.B. in verschiedener Färbung und Gestalt, zusammenstellen.

5. Die in der bevorzugten Ausführungsform Schwarzbrot (Vollkornbrot) ähnlich sehende äussere Form des Präparates sowie der Umstand, dass das Mittel ausreichend gekaut werden muss, simulieren einen ausgesprochenen Nahrungsmittelcharakter. Dieser Nahrungsmittelcharakter erhöht wesentlich die Akzeptanz des Präparates für den Verbraucher.

6. Die besondere galenische Entwicklungsform der Präparation bringt weitere Vorteile für die Einnahme mit sich; die pulverförmige bzw. faserige Struktur der Pektin- bzw. Kleiekomponente bedingt eine ausgesprochen ungünstige Einnahmemöglichkeit. Durch den Gelatineanteil wird die Schluckfähigkeit des Präparates eindeutig verbessert, wenn nicht sogar erst ermöglicht. Durch hinreichend langes Verrühren mit der flüssigen Gelatine während des Produktionsprozesses werden die Faseranteile mit einem Proteinmantel versehen, wodurch verhindert wird, dass die Faseranteile sich in der Mundhöhle (Zähne, Schleimhautduplikaturen) festsetzen oder gar durch Reizung des Rachenringes zu Hustenanfällen führen.

7. Überraschenderweise traten im Verlauf einer Freiwilligenstudie an 6 Probanden während der gesamten Versuchsdauer nicht die literaturbekannten Eigenschaften des Pektins, wie Magenknurren oder Darmkollern auf, was ebenfalls dem Einfluss von Gelatine zugeordnet werden muss. Weiter überraschend trat auch eine deutliche Beeinflussung des Verhältnisses von β- zu α-Serumlipoprotein im Sinne einer gewünschten Senkung auf.

Das erfindungsgemässe Diätmittel weist einen niedrigen metabolen Brennwert auf und unterdrückt überraschend wirkungsvoll das natürlich auftretende Hungergefühl, wenn es als Ersatz von Hauptmahlzeiten angewendet wird. Hunger wird bekanntlich grundsätzlich von zwei körperlichen bzw. physiologischen Faktoren bestimmt, nämlich (a) einem Absinken der Spannung (Tonus) der Magendarmwand in Abhängigkeit vom Füllungszustand und (b) einem Absinken des Blutglukosespiegels unter Werte von 80 mg/100 ml. Durch den aus der Kleie und dem Pektin stammenden hohen Gehalt des erfindungsgemässen Diätmittels an unverdaulichen pflanzlichen Faserstoffen bleibt der Füllungszustand des Magendarmkanals auch während der Verdauungsvorgänge zufriedenstellend. Zusätzlich wird die Wandspannung im Magendarmtrakt aber noch durch die Gelatine günstig beeinflusst, die einen besonderen Quelleffekt aufweist. Die Gelatine bewirkt andererseits, dass der Blutglukosespiegel nicht (über längere Zeit anhaltend) unter 80 mg/100 ml sinkt.

Das im erfindungsgemässen Diätmittel enthaltene Pektin bewirkt durch eine Bindung von Cholesterin, welches im Darm vorhanden ist, eine Senkung des Serumcholesterinspiegels, indem es, da selbst unverdaulich, dessen Resorption verhindert und die Ausscheidung fördert. Dieses im Darm vorhandene Cholesterin stammt dabei nur zum geringeren Teil aus der direkt zugeführten Nahrung, sondern gelangt vorzugsweise über die Gallenflüssigkeit in den Zwölffingerdarm, um dann normalerweise im Jejunum wieder rückresorbiert zu werden. Vor allem durch die Unterbrechung dieses sogenannten enterohepatischen Kreislaufs wird der Serumspiegel des Cholesterins gesenkt.

Bekanntlich besteht eine der wesentlichen Aufgaben der aus der Nahrung stammenden Pflanzenfasern in der Bindung weiterer natürlicher Stoffwechselprodukte, die im Darminhalt vorhanden sind, und deren Ausscheidung mit dem Stuhl. In diesem Zusammenhang wurde in epidermiologischen Studien in den vergangenen Jahren ein sprunghaftes Ansteigen der Fälle von Darmkrebs in westlichen Ländern gezeigt, was auf dort geänderte Essgewohnheiten zurückgeführt wurde, wobei der verringerte Anteil an pflanzlichen Fasern als kausale Ursache vermutet wurde. So ist in bestimmten Ländern der 3. Welt mit einem sehr hohen Faseranteil in der Nahrung Darmkrebs deutlich seltener. Auch das klinische Syndrom der Darmdivertikulose, das in westlichen Ländern vermehrt diagnostiziert wird, soll in einem direkten Zusammenhang zum verminderten Fasergehalt der Nahrung stehen.

Die Motorik des Magendarmkanals und hierüber sein gesamter Funktionszustand wird über einen Füllungszustand mitbestimmt. Hierbei spielt der Gehalt der Nahrung an unverdaulichen Resten (Ballaststoffe) eine wichtige Rolle. Diese Motorik, unter welcher auch die Peristaltik der Gallenblase mitzubetrachten ist, ist für den gesamten Stoffaustausch im Magendarmbereich entscheidend und bestimmt das Ausmass von Resorption, Sekretion und Defäkation. Störungen dieser Abläufe führen zu chronischen Entzündungen der Darmwand und mithin auch zu Darmkrebs.

Das erfindungsgemässe Diätmittel bewirkt also eine Verringerung des Körpergewichts und eine Senkung des Serumcholesterinspiegels, ohne dass es – wie bei vielen üblichen «Hungerkuren» – zu einer Störung der natürlichen Verdauungsvorgänge kommt.

Bei der Herstellung des erfindungsgemässen Diätmittels löst man zunächst Gelatine und Pektin in wenig Wasser, verteilt sodann die Kleie in der viskosen Lösung und verarbeitet schliesslich die Masse zu beliebigen Formkörpern, beispielsweise Würfeln, Platten oder Granulat.

Als Gelatinebestandteil des erfindungsgemässen Diätmittels kann jede Speisegelatine, die aus Haut oder Knochen gewonnen wurde, eingesetzt werden. Bevorzugt wird eine Haut- oder Knochengelatine, insbesondere Hautgelatine, die in 1%iger Lösung bei 37°C eine Viskosität von 3 bis 10 mPas aufweist und ein mittleres Molekularge-

wicht von 40 000 bis 60 000 Dalton besitzt. Die Gallertfestigkeit der Gelatine sollte vorzugsweise bei 100 bis 300 Bloomgramm liegen.

Als Pektinbestandteil kann jedes Obst- oder Rübenpektin mit Lebensmittelqualität eingesetzt werden. Vorzugsweise wird ein Obstpektin, z.B. Citrus- oder Apfelpektin, verwendet.

Citruspektin besteht praktisch aus partiell methylveresterter Polygalakturonsäure. Besonders bevorzugt wird Apfelpektin eingesetzt. Brauchbare Pektine weisen mittlere Molekulargewichte von 60 000 bis 90 000 Dalton auf.

Als Kleiebestandteil des erfindungsgemässen Diätmittels kann jedes beim Mahlen von Getreide als Nebenprodukt angefallene Kleieprodukt, das die zerkleinerten Bestandteile der äusseren Getreidekornschichten enthält, verwendet werden. Besonders bevorzugt wird Weizenkleie. Brauchbare Kleieprodukte können z.B. aus 21% Zellulose, 20 bis 26% Pentosanen (Hemizellulosen); 7,5 bis 9% Stärke, 5% Zucker, 11 bis 15% Proteinen, 5 bis 10% Fett, 5 bis 9% Asche und 14% Wasser bestehen. Sie sollten möglichst keimarm oder sogar steril sein.

Das Gewichtsverhältnis von Gelatine zu Pektin zu Kleie beträgt vorzugsweise 100:5:80 bis 100:43:28.

Nach einer bevorzugten Ausführungsform enthält das erfindungsgemässe Diätmittel, wenn es anstelle einer Mahlzeit über eine längere Dauer eingesetzt werden soll, zusätzlich hochwertiges tierisches Eiweiss oder diesem biologisch gleichwertiges Eiweiss. Ein besonders brauchbares Beispiel für ein hochwertiges tierisches Eiweiss ist Milcheiweisspulver. Weitere Beispiele sind Eipulver, proteinhaltiger Fleischextrakt oder tierische Serumproteine. Der Gehalt des Diätmittels an dem hochwertigen tierischen Eiweiss kann z.B. 50% betragen. Vorzugsweise beträgt das Gewichtsverhältnis von Gelatine zu Pektin zu Kleie zu hochwertigem Eiweiss 100:15:75:250 bis 100:25:150:400, insbesondere 100:15:100:250.

Wenn das erfindungsgemässe Diätmittel anstelle einer Mahlzeit eingesetzt wird, sollte es auch eine geringe Menge, beispielsweise 5 bis 7%, insbesondere 6%, an essentiellen Fettsäuren, wie Linolsäure oder Ölsäure, sowie Vitamine und Mineralsalze enthalten. Als Vitamine sollte es z.B. Ascorbinsäure (Vitamin C), Vitamin A, die Vitamine $B_1$, $B_2$, $B_6$ und $B_{12}$, die Vitamine $D_2$ und $D_3$, Vitamin E und Vitamin K enthalten. Als Mineralsalze sind Calcium- und Eisensalze besonders wichtig.

Das erfindungsgemässe Diätmittel kann zusätzlich Geschmacksstoffe enthalten. Als derartige zusätzliche Geschmacksstoffe eignen sich insbesondere Süssstoffe, wie Saccharin und dessen Salze oder Cyclamat, Fruchtsäuren, wie Zitronensäure oder Äpfelsäure, Fruchtaromen, wie Orangenaroma, Nougat- oder Haselnussaroma, oder auch ein Hefeextrakt, der einen Fleischgeschmack vermittelt, oder ein sogenanntes «Schweinekrusten-Aroma», das einen Speckgeschmack vermittelt, oder auch sonstige Gewürze, wie beispielsweise Zwiebel-, Sellerie-, Tomaten-, Curry-, Kaffee-, Pilz-, Kakao- oder Kräuterpulver.

Auch Farbstoffe mit Lebensmittelqualität können dem erfindungsgemässen Diätmittel zugesetzt werden, um es für den Verbraucher attraktiver und je nach dem zugesetzten Geschmackstoff einem natürlichen Lebensmittel ähnlicher zu machen.

Weitere mögliche zusätzliche Bestandteile des erfindungsgemässen Diätmittels sind Konservierungsmittel, wie beispielsweise Weinsäure oder andere Fruchtsäuren, Kaliumsorbat, Benzoesäure, Ameisensäure, Schwefeldioxid, schweflige Säure, Natriumsulfit oder Hexamethylentetramin. Der Zusatz von Fruchtsäuren ist aus geschmacklichen Gründen begrenzt. Ein pH-Wert von 4 im Endprodukt sollte möglichst nicht unterschritten werden.

Das erfindungsgemässe Diätmittel kann auch zusätzlich galenische Hilfsmittel, insbesondere Tablettierungshilfen, enthalten.

Bei der Verarbeitung der breiigen Masse zu beliebigen Formkörpern, z.B. Presslinge, Würfel, Platten, Granulat, Dragees oder auch Kapselfüllungen werden an sich bekannte Verfahren angewendet.

So kann die Masse beispielsweise stranggepresst oder auf einem Kühlband ausgewalzt und nach dem Erstarren zu Platten oder Würfeln zerschnitten werden. Sie kann aber auch mit Hilfe einer Granulierwalze oder durch Pelletisieren oder Sprühgranulation zu Granulat verarbeitet werden.

Nach einer bevorzugten Ausführungsform kann die breiige Masse in Schichten von 0,1 bis 1 mm Dicke scharf getrocknet, in schuppige Täfelchen gebrochen und dann zu mundgerechten Presslingen geformt werden.

Nach einer weiteren bevorzugten Ausführungsform kann man die Formkörper, beispielsweise mundgerechte Stücke, gegebenenfalls nach Aufschäumen, einfrieren und einer Gefriertrocknung unterwerfen, wobei lockere, knusprige und ohne Konservierungsschutz lagerstabile Stückchen erhalten werden. Es war überraschend, festzustellen, dass die Gelatine beim Einfrieren sich nicht entmischt und nach Wiederauftauen ausläuft.

Nach einer anderen bevorzugten Ausführungsform wird die breiige Masse zu Haarnudelsträngen extrudiert, die zu mundgerechten Häppchen aufgewickelt bzw. «versponnen» und dann scharf getrocknet werden.

Um die Verwendung von Konservierungsmitteln zu vermeiden, kann die Herstellung auch unter völlig aseptischen Bedingungen durchgeführt werden, wobei anschliessend eine entsprechende Verpackung erfolgt, die absolut sicheren Schutz gegenüber bakterieller Kontamination bietet, z.B. eine Einsiegelung unter Vakuum oder unter Verwendung von Kohlensäure als Schutzgas. Auch Bestrahlung mit γ-Strahlen kommt in Frage.

In einer orientierenden Pilotstudie, in der 6 Probanden täglich zwischen 60–90 g des Diätmittels nach Beispiel 1 zusammen mit einer standardisierten Zusatzmahlzeit über 12 Tage hinweg einnahmen, wurde ein durchschnittlicher Gewichtsverlust von 4,8 kg pro Teilnehmer erreicht (3–6,5 kg). Gleichzeitig kam es zu einer Reduktion sowohl des Serumcholesterin um durchschnittlich 14% des Ausgangswertes als auch des Serumtriglyceridspiegels um durchschnittlich 44%. Unerwartet war auch eine deutliche Beeinflussung des Verhältnisses von β- zu α-Serumlipoprotein im Sinne einer Senkung. Einem derartigen Befund wird nach der derzeitigen medizinischen Lehrmeinung ein hoher therapeutischer bzw. prophylaktischer Nutzen bei der Beeinflussung der o.g. angiologischen Erkrankungen zugeordnet. Ausserdem wurde von allen Probanden während der 12tägigen Anwendung keinerlei Beeinträchtigung der natürlichen Verdauungsprozesse beobachtet, wie überhaupt die Verträglichkeit des Mittels übereinstimmend als sehr gut beurteilt wurde. Überraschend war auch das Ausbleiben von anhaltendem oder unerträglichem Hungergefühl während der Studie. Die Einnahme von 10 bis 20 g Präparat alle 2 Stunden verhindert dabei weitgehend «Spitzen» von Hungergefühl.

Das erfindungsgemässe Diätmittel kann selbstverständlich sowohl als Nahrungsmittel als auch als Heilmittel, z.B. in Form von Kautabletten, eingesetzt werden.

Die folgenden Beispiele erläutern die Erfindung:

Beispiel 1
Es wurde ein Diätmittel mit folgender Zusammensetzung hergestellt:

| | |
|---|---|
| 2 800 g | keimarme Weizenkleie |
| 1 400 g | Apfelpektin |
| 7 000 g | Hautgelatine vom Rind mit einer Gallertfestigkeit von 190 Bloomgramm und einer Teilchengrösse von 4 mm (grob) |
| 400 g | pulverisierte «gebackene Schweinekrusten» (Marke «Snäcky» der Fa. G. Reimer & co., Limburg/Lahn) |
| 4,5 l | Wasser |
| 12,9 g | Kaliumsorbat |
| 16 112,9 g | Gesamtgewicht |

Zur Herstellung liess man die Gelatine in kaltem Wasser etwa 1 Stunde vorquellen, erhitzte dann den Ansatz auf etwa 65°C und brachte so die Gelatine in Lösung. Man erhielt eine hochviskose Flüssigkeit, in die alle weiteren Zusätze hineingegeben wurden. Durch Rühren wurden die Zusätze in der Gelatinelösung verteilt. Dies erforderte einige Zeit, da das Gemisch eine zähe, breiige Konsistenz aufwies. Das Substanzgemisch wurde dann auf einem Kühlband ausgewalzt und durch Abkühlung zum Erstarren gebracht. Nach erfolgter Erstarrung wurde die Substanzschicht durch Schneiden zu Platten mit einem Gewicht von jeweils 10 g portioniert und verpackt.

Beispiel 2
Nach der Arbeitsweise von Beispiel 1 wurde ein Diätmittel mit folgender Zusammensetzung hergestellt:

| | |
|---|---|
| 35 g | Hautgelatine mit einer Gallertfestigkeit von 190 Bloomgramm und einer Teilchengrösse bis 4 mm (grob) |
| 5 g | Citruspektin |
| 20 g | keimarme Weizenkleie |
| 30 g | Wasser |
| 5 g | Weinsäure |
| 1 g | Ascorbinsäure |
| 8 ml | flüssiges «Assugrin»® (Saccharin und Cyclamat) |
| 0,2 ml | natürliches Orangen-Aroma |
| 0,04 g | Kaliumsorbat |
| 104,24 g | Gesamtgewicht |

Das Endprodukt hatte einen pH-Wert von etwa 4,0 und einen Wassergehalt von etwa 40%.

Beispiel 3
Nach der Arbeitsweise von Beispiel 1 wurde ein Diätmittel mit folgender Zusammensetzung hergestellt:

| | |
|---|---|
| 35 g | Hautgelatine |
| 3 g | Apfelpektin |
| 30 g | sterile Weizenkleie |
| 30 g | Wasser |
| 5 g | Weinsäure |
| 8 g | Saccharin |
| 0,2 ml | natürliches Orangen-Aroma |
| 0,04 g | Kaliumsorbat |
| 111,24 g | Gesamtgewicht |

Beispiel 4
Die Arbeitsweise von Beispiel 2 wurde wiederholt, wobei jedoch in der Zusammensetzung anstelle von 5 g Citruspektin 15,0 g Apfelpektin und anstelle von 20 g Weizenkleie 10,0 g Weizenkleie eingesetzt wurden.

Beispiel 5
Die Arbeitsweise von Beispiel 2 wurde wiederholt, wobei jedoch in der Zusammensetzung anstelle von 5 g Citruspektin 12,5 g Apfelpektin und anstelle von 20 g Weizenkleie 12,5 g Weizenkleie eingestezt wurden.

Beispiel 6
Die Arbeitsweise von Beispiel 2 wurde wiederholt, wobei jedoch in der Zusammensetzung anstelle von 5 g Citruspektin 15 g Apfelpektin eingesetzt wurden.

Beispiel 7
2000 g Weizenkleie wurden mit 1000 g Apfelpektin und 290 g pulverisierten «gebackenen Schweinekrusten» (vgl. Beispiel 1) innig vermischt und in eine Lösung von 4600 g Hautgelatine (190 Bloom) und 10,5 g Kaliumsorbat in 5,3 l Wasser bei 65°C eingerührt.

Die homogenisierte zähflüssige Masse wurde auf einem Kühlband ausgewalzt und zum Erstarren gebracht. Nach Zerschneiden der Happen in quadrige Häppchen von h × b × l wie 1 cm × 2 cm × 5 cm wurde in Polyethylen eingesiegelt und in tiefgefrorenem Zustand aufbewahrt. Die aufgetauten Häppchen wurden durch Zerkauen verzehrt.

Wiedereinfrieren zur Langzeitaufbewahrung in hermetisch verschlossenen Gefässen ergab keinerlei Qualitätsminderung.

## Beispiel 8

Eine Aufschlämmung von 15 g Weizenkleie in einem Gemisch von 7,5 g Apfelpektin und 37,5 g Rinderhaut-Gelatine, in 540 ml Wasser bei 70°C gelöst, wurde in der Küchenmaschine mit dem Schneebesen-Rührwerk schaumig geschlagen. Dann wurde die in etwa 1,5 cm Schichthöhe ausgegossene Masse gekühlt und in 2 cm breite Streifen geschnitten. Die Streifen wurden im Gefrierschrank bei -18°C eingefroren und anschliessend gefriergetrocknet.

Die lockeren knusprigen Häppchen wiesen einen knäckebrotähnlichen Geschmack auf.

## Beispiel 9

In einer gemäss Beispiel 8 hergestellten Masse wurden vor dem Schaumigschlagen 250 g pürierte Himbeeren und 5 ml flüssiges «Natreen»® eingerührt.

Nach analoger Verarbeitung ergaben sich rötlich gefärbte, fruchtig schmeckende Häppchen, die an der Luft liegend ohne Veränderungen ein halbes Jahr aufbewahrt wurden.

## Beispiel 10

In eine gemäss Beispiel 8 hergestellte Masse wurden vor dem Schaumigschlagen 8 g Neuform-Hefeextrakt-Kräuterpaste «Vitam-R®» (Vitam GmbH D-3250 Hameln) eingerührt. Die analog wie in Beispiel 8 hergestellten Häppchen wiesen einen fleischähnlichen Geschmack nach Bratensosse auf.

## Beispiel 11

Die Aromatisierung der gemäss Beispiel 8 hergestellten 600 g Grundmasse erfolgte mit 60 g pulverisierten «gebackenen Schweinekrusten» (vgl. Beispiel 1). Die gefriergetrockneten Häppchen fanden mit ihrem intensiven Speckgeschmack ohne Fettgehalt besonders grossen Anklang bei Probanden.

## Beispiel 12

Die Arbeitsweise von Beispiel 8 wurde wiederholt, wobei jedoch in der Zusammensetzung 30 g Weizenkleie anstelle von 15 g, 2 g Apfelpektin anstelle von 7,5 g und 600 ml Wasser anstelle von 540 ml unter Beibehaltung des Gelatinegehaltes von 37,5 g eingesetzt wurden.

## Beispiel 13

Es wurde ein Diätmittel aus den folgenden Bestandteilen hergestellt:

| | |
|---|---|
| 50 g | Milcheiweiss |
| 20 g | keimarme Weizenkleie |
| 20 g | Hautgelatine vom Rind mit einer Gallertfestigkeit von 190 Bloomgramm, feingemahlen |
| 3 g | Apfelpektin |
| 2 g | Ölgemisch aus Sojaöl/Distelöl |
| 1,8 g | Vitamin-Mineralsalz-Gemisch (enthaltend die Vitamine A, $B_1$, $B_2$, $B_6$, C, D und E, Calcium und Eisen) |
| 0,3 g | Nougat-Aroma |
| | Wasser und Tablettierhilfe auf 100 g |

Die Bestandteile wurden zu einem rieselfähigen Gemisch vermischt und zu Tabletten verpresst.

## Patentansprüche

1. Diätmittel, das zur Verringerung des Körpergewichts und Senkung des Serumlipidspiegels geeignet ist, enthaltend Pektin und Kleie, dadurch gekennzeichnet, dass es neben Pektin und Kleie Gelatine enthält.

2. Diätmittel nach Anspruch 1, dadurch gekennzeichnet, dass es zusätzlich hochwertiges tierisches Eiweiss oder diesem biologisch gleichwertiges Eiweiss enthält.

3. Diätmittel nach Anspruch 1 oder 2, dadurch gekennzeichnet, dass es zusätzlich essentielle Fettsäuren, Vitamine und/oder Mineralsalze enthält.

4. Diätmittel nach einem der Ansprüche 1 bis 3, dadurch gekennzeichnet, dass es zusätzlich Geschmacks-, Farb- und/oder Konservierungsstoffe sowie galenische Hilfsmittel, insbesondere Tablettierungshilfen, enthält.

5. Diätmittel nach Anspruch 1, dadurch gekennzeichnet, dass das Gewichtsverhältnis von Gelatine zu Pektin zu Kleie 100:5:80 bis 100:43:28 beträgt.

6. Diätmittel nach Anspruch 2, dadurch gekennzeichnet, dass das Gewichtsverhältnis von Gelatine zu Pektin zu Kleie zu hochwertigem Eiweiss 100:15:75:250 bis 100:25:150:400, vorzugsweise 100:15:100:250 beträgt.

7. Diätmittel nach einem der Ansprüche 1 bis 6, dadurch gekennzeichnet, dass es als trockener Schaum, Granulat, Fasergespinst, Pressling, Dragee, Kapselfüllung oder als feuchte Masse in Form beliebiger Formkörper vorliegt.

8. Diätmittel nach einem der Ansprüche 1 bis 7, dadurch gekennzeichnet, dass es als Pektin Citruspektin oder Apfelpektin enthält.

9. Diätmittel nach einem der Ansprüche 1 bis 8, dadurch gekennzeichnet, dass es als Kleie Weizenkleie enthält.

10. Diätmittel nach einem der Ansprüche 1 bis 9, dadurch gekennzeichnet, dass es als Gelatine eine Hautgelatine enthält.

11. Verfahren zur Herstellung des Diätmittels nach einem der Ansprüche 1 bis 10, dadurch gekennzeichnet, dass man Gelatine und Pektin in wenig Wasser löst, in der viskosen Lösung die Kleie und gegebenenfalls die weiteren Zusatzstoffe verteilt und die breiige Masse zu beliebigen Formkörpern verarbeitet.

12. Verfahren nach Anspruch 11, dadurch gekennzeichnet, dass man die Formkörper, gegebenenfalls nach Aufschäumen, einfriert und durch Gefriertrocknung zu lockeren knusprigen Stückchen verarbeitet.

13. Verfahren nach Anspruch 11, dadurch gekennzeichnet, dass man die breiige Masse zu Haarnudelsträngen extrudiert, zu mundgerechten Häppchen aufwickelt und scharf trocknet.

14. Verfahren nach Anspruch 11, dadurch gekennzeichnet, dass man die breiige Masse in Schichten von 0,1 bis 1 mm Dicke scharf trocknet, in schuppige Täfelchen bricht und dann zu mundgerechten Presslingen formt.

## Claims

1. Diet composition, suitable for reduction of body weight and lowering of the serum lipid level, containing pectin and bran, characterized in containing besides pectin and bran gelatin.

2. Diet composition according to claim 1, characterized in containing additionally high-grade animal protein or protein being biologically equivalent thereto.

3. Diet composition according to claims 1 and 2, characterized in containing additionally essential fatty acids, vitamins and/or mineral salts.

4. Diet composition according to one of claims 1 to 3, characterized in containing additionally flavours, colours and/or preservatives as well as galenic auxiliaries, particularly tabletting aids.

5. Diet composition according to claim 1, characterized in the weight ratio of gelatin to pectin to bran ranging from 100:5:80 to 100:43:28.

6. Diet composition according to claim 2, characterized in the weight ratio of gelatin to pectin to bran to high-grade protein ranging from 100:15:75:250 to 100:25:150:400, preferably 100:15:100:250.

7. Diet composition according to one of claims 1 to 6, characterized in it being present as dry foam, granulate, fibrous web, pressed article, dragee, capsule filling or a moist mass in the form of randomly molded articles.

8. Diet composition according to one of claims 1 to 7, characterized in the pectin being citrus pectin or apple pectin.

9. Diet composition according to one of claims 1 to 8, characterized in the bran being wheat bran.

10. Diet composition according to one of claims 1 to 9, characterized in the gelatin being a skin gelatin.

11. Process for the preparation of the diet compositon according to one of claims 1 to 10, characterized in diluting gelatin and pectin in little water, dispersing the bran and, if desired, the further additives in the viscous solution, and preparing randomly molded articles from the pulpy mass.

12. Process according to claim 11, characterized in freezing the molded articles, if desired after foaming-up, and preparing fluffy crisp pieces by freeze-drying.

13. Process according to claim 11, characterized in extruding the pulpy mass to spaghetti-like strands, winding them up to bite-sized pieces and drying them rapidly.

14. Process according to claim 11, characterized in rapidly drying the pulpy mass in layers of 0.1 to 1 mm thickness, breaking them into flaky tablets and then forming them into bite-sized molded articles.

## Revendications

1. Produit diététique, prévu pour la diminution du poids et la réduction du niveau des lipides sériques, contenant de la pectine et du son, caractérisé par le fait qu'il contient outre le pectine et le son de la gélatine.

2. Produit diététique selon la revendication 1, caractérisé par le fait qu'il contient en plus de la protéine animale à haute valeur ou une protéine biologiquement comparable.

3. Produit diététique selon la revendication 1 ou 2, caractérisé par le fait qu'il contient en plus des acides gras essentiels, des vitamines et/ou des sels minéraux.

4. Produit diététique selon l'une des revendications 1 à 3, caractérisé par le fait qu'il contient en plus des substances gustatives, colorantes et/ou de conservation ainsi que des additifs galéniques, en particulier additifs pour former des comprimés.

5. Produit diététique selon la revendication 1, caractérisé par le fait que la proportion de poids entre la gélatine, la pectine et le son est de 100:5:80 à 100:43:28.

6. Produit diététique selon la revendication 2, caractérisé par le fait que la proportion de poids entre la gélatine, la pectine, le son et la protéine à haute valeur est de 100:15:75:250 à 100:25:150:400, de préférence 100:15:100:250.

7. Produit diététique selon l'une des revendications 1 à 6, caractérisé par le fait qu'il apparaît comme mousse sèche, granulé, matière fibreuse, matière pressée, dragée, contenu de capsule ou comme une masse humide qui peut être modelée à souhait.

8. Produit diététique selon l'une des revendications 1 à 7, caractérisé par le fait qu'il contient comme pectine de la pectine d'agrumes ou de la pectine de pommes.

9. Produit diététique selon l'une des revendications 1 à 8, caractérisé par le fait qu'il contient comme son du son de blé.

10. Produit diététique selon l'une des revendications 1 à 9, caractérisé par le fait qu'il contient comme gélatine une gélatine provenant de la peau.

11. Procédé de production du produit diététique selon l'une des revendications 1 à 10, caractérisé par le fait que l'on dissout dans un peu d'eau la gélatine et la pectine, que l'on répand dans la solution visqueuse le son et éventuellement les autres substances et que l'on modèle la bouillie à souhait.

12. Procédé selon la revendication 11, caractérisé par le fait que l'on congèle les pièces façonnées, éventuellement après formation de mousse, et que l'on obtient par lypophilisation de petits morceux légers et croustillants.

13. Procédé selon la revendication 11, caractérisé par le fait que l'on forme à partir de la bouillie des vermicelles, qui sont alors enroulés en petites bouchées convenable à la bouche et qui sont séchés intensivement.

14. Procédé selon la revendication 11, caractérisé par le fait que l'on sèche intensivement la masse en bouillie en couches de 0,1 à 1 mm d'épaisseur, qu'on les casse en petites plaques écailleuses qui sont alors façonnées en pièces moulées convenable à la bouche.